Europäisches Patentamt

European Patent Office

Office européen des brevets

(19)

(11) Publication number: **0 168 008**
**A2**

## (12) EUROPEAN PATENT APPLICATION

(21) Application number: 85108409.5

(22) Date of filing: 06.07.85

(51) Int. Cl.⁴: **A 61 K 45/02**
**//C12N15/00, C12P21/02**

(30) Priority: 10.07.84 PC T/JP84/00352
12.04.85 PC T/JP85/00190

(43) Date of publication of application:
15.01.86 Bulletin 86/3

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Akagi, Yasaburo
18-18, Matsugaoka 3-chome
Takatsuki-shi Osaka 569(JP)

(72) Inventor: Miura, Yasumoto
4-43, Seiwadainishi 2-chome
Kawanishi-shi Hyogo 666-01(JP)

(72) Inventor: Hoshino, Tetsuo
13-37, Terauchi 2-chome
Toyonaka-shi Osaka 560(JP)

(74) Representative: von Kreisler, Alek, Dipl.-Chem. et al,
Deichmannhaus am Hauptbahnhof
D-5000 Köln 1(DE)

(54) Stable composition of gamma-interferon.

(57) The present invention relates to a human γ-interferon composition frozen or lyophilized in the substantial absence of inorganic salt and in the presence of amino acid.

The activity of the above composition decreases very little during freezing or lyophilization and during storage. Upon thawing or reconstitution of the composition, no turbidity is produced. Therefore, the composition can advantageously be used as an antiviral or antitumor agent, among others.

EP 0 168 008 A2

Croydon Printing Company Ltd.

Stable Composition of γ-Inteferon

This invention relates to human γ-interferon composition.

Interferons are at present classified into three types, α, β and γ. α- and β-interferons are relatively stable and have been submitted to clinical trials mostly in the dosage form to be suited for parenteral administration. Systematic study has also progressed to a considerable extent. On the other hand, type γ interferon (hereinafter sometimes abbreviated as IFN-γ) is very unstable and its aqueous solution easily decreases in IFN-γ activity during storage or during the process of freezing or lyophilization. Another difficulty is that when lyophilization products are reconstituted, turbidity is observed in the resulting solutions. These and other difficulties have made it difficult to prepare stable compositions worth clinical use and, as a result, have much retarded the clinical application of IFN-γ that is very potent in antiviral activity or antitumor activity [Salvin et al., Journal of The National Cancer Institute, 55, 1233 (1975)] among interferons and is most expected to serve as a drug.

The interferon bulk substance to be submitted for use in pharmaceutical preparation is generally obtained in highly pure state starting from crude interferon and after an isolation/purification process involving a variety of chromatographic techniques, among others. In said isolation/purification process, various inorganic and organic compounds are used. Thus, for instance, inorganic ions used for pH and ion strength adjustment occur in the purified aqueous interferon solution. It has so far been considered that said inorganic ions (inorganic salts) might advantageously serve as stabilizers or the like in the process of pharmaceutical preparation.

For instance, there is a disclosure that nonglycocylated-β-interferon can be stabilized by addition of an inorganic salt (Japanese Unexamined Patent Publication No. 25364/1984).

With such technological background, the present inventors unexpectedly found that when aqueous IFN-γ solutions with decreased inorganic salt concentrations are used in dosage form preparation, the decrease in IFN-γ activity during the freezing and lyophilization processes becomes still less as compared with the inorganic salt-containing prior art aqueous solution of IFN-γ and that there can be obtained a stable IFN-γ composition which produces no turbidity upon reconstitution. These findings and continued study have now led to completion of the present invention.

The present invention provides a human γ-interferon composition frozen or lyophilized in the substantial absence of inorganic salt and in the presence of amino acid.

The IFN-γ to be used in the practice of the invention

0168008

may be any human-derived IFN-γ, whether it is a natural one (nIFN-γ) or one obtained by recombinant DNA technology(rIFN-γ).

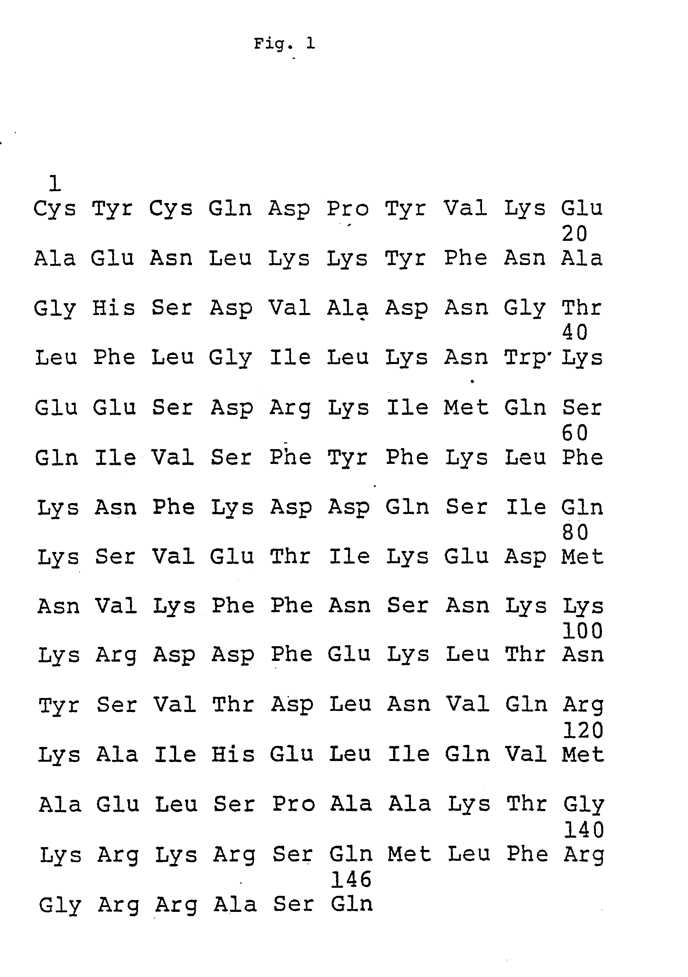

More concretely, the above-mentioned rIFN-γ includes polypeptide consisting of 146 amino acids, for example, of the sequence shown in Figure 1 and various fragments of the polypeptide, such as N terminal portion-deficient species, i.e. lacking not more than four amino acids of the N terminal part of the polypeptide and C terminal portion-deficient species which are cleaved at a site not earlier than the 131st amino acid residue of the polypeptide or the N terminal portion-deficient species. Furthermore, the rIFN-γ includes a polypeptide the cystein residues of the polypeptide being replaced by serine or threonine.

The polypeptide consisting 146 amino acid shown in Figure 1 and $Cys^1$-$Tyr^2$-$Cys^3$-deficient species of the polypeptide [des($Cys^1$-$Tyr^2$-$Cys^3$)IFN-γ] are preferred among others.

Further more, the use of an aqueous solution containing IFN-γ obtained by the gene recombinant technique (rIFN-γ) in a high concentration is advantageous.

The human IFN-γ preferably has a specific activity of $1 \times 10^5$ to $1 \times 10^7$ international units/mg (IU/mg) and the aqueous solution of IFN-γ preferably has an activity of $1 \times 10^2$ to $1 \times 10^7$ IU/ml, more preferably $1 \times 10^4$ to $1 \times 10^7$ IU/ml.

The above aqueous IFN-γ solution should be substantially free of inorganic salts. As used herein, "substantially free" means that the inorganic salt concentration is not more than 0.1 M, preferably not more than 0.05 M, more preferably not more than 1 mM. As regards the inorganic ion strength, the ion strength calculated on the basis of inorganic ion concentrations should be not more than 0.1, preferably not more than 0.05, more preferably not more than 0.001. Furthermore, for the lyophilized composition, the inorganic salt content should be not more than 30%, preferably not more than 15%, more preferably not more than 0.3%, based on the whole weight of the composition.

The substantially inorganic salt-free aqueous solution of IFN-γ can be produced, for example by using an inorganic salt-free buffer as the buffer for use in the IFN-γ purification process, in particular in the final-step chromatography, or by removing inorganic salts from a purified aqueous solution of IFN-γ.

Preferred as the amino acid is a monoamino aliphatic amino acid such as glycine, α-alanine, β-alanine, leucine, glutamic acid or aspartic acid, in particular glycine. Physiologically acceptable salts or derivatives of such amino acid may also be used. These amino acids may be used singly or in combination of two or more. The amino acid to be used may be of commercial grade. For clinical application of the composition of the invention, however, the amino acid should preferably be of such grade as can be adequate for parenteral administration.

The total amino acid amount is preferably not less than 1 mg, more preferably 5-50 mg, per ml of aqueous IFN-γ solution.

The composition of the invention may, in cases where the composition contains cysteine residue, also contain a reducing sulfur compound such as glutathione (reduced form), thioctic acid, thiodiglycol, thioethanolamine, monothioglycerol, dithiothreitol or a thioalkanoic acid containing 1-7 carbon atoms, in particular glutathione (reduced form). The reducing sulfur compound, when used, is used in an amount of not less than 0.1 mg, preferably 0.5-10 mg, per ml of aqueous IFN-γ solution.

Furthermore, human serum albumin (HSA) and/or a sugar may be added as a further stabilizing agent. The HSA may be of any grade but, for the clinical use of the composition according to the invention, the parenteral administration grade or the like is preferred. For instance, there may be used that HSA produced from normal human plasma as raw material by fractionation and purification by Cohn's sixth ethanol fractionation method [Journal of the Anerican Chemical Society, volume 68, pages 459-475 (1946)]. In addition, the HSA may contain acetyltryptophan sodium or sodium caprylate as a stabilizer.

HSA is preferably used in an amount of not less than 1 mg, more preferably 2 to 20 mg, per milliliter of aqueous IFN-γ solution.

In cases where the composition according to the invention contains HSA, it is preferable to adjust the composition such that when in its solution state, it gives a pH of 4.0 to 5.0 or 7.5 to 8.5. More particularly, when the above-mentioned amino acid is a neutral amino acid, it is preferable to adjust the pH to 4.0 to 5.0, while, when said amino acid is an acidic amino acid, the pH is preferably adjusted to 7.5 to 8.5.

As a sugar, one or more substances selected from among sugars, for example polysaccharides such as dextran and hydroxyethylstarch, disaccharides such as sucrose, maltose and lactose and monosaccharides such as glucose, fructose and galactose, may also be exemplified.

Referring to the dextran and hydroxyethylstarch mentioned above, they may be of commercial grade. For clinical application of the composition of the invention, however, they should preferably be of such grade as can be suited for parenteral administration as plasma substitutes. It is advantageous to use a dextran having an average molecular weight of 10,000 to 100,000, preferably 40,000 to 70,000 or a hydroxyethylstarch having an average molecular weight of 10,000 to 200,000, preferably 20,000 to 60,000 or 200,000.

The above sugars, when added, are added in an amount of not less than 1 mg, preferably 3 mg to 50 mg, per ml of aqueous IFN-γ solution.

Whereas the present composition may contain the above-mentioned sugar and/or amino acid or the like as an osmotic pressure modifier, the addition of an inorganic salt such as sodium chloride is disagreeable since it rather deteriorates the quality of the composition. The above-mentioned preferable osmotic pressure modifier may be added to said composition in advance or added to a solvent for redissolving the lyophilizate.

The frozen and lyophilized human IFN-γ compositions according to the invention can be produced, for example, in the following manner.

To a substantially inorganic salt-free aqueous solu-

tion containing $1 \times 10^2$ to $1 \times 10^7$ IU/ml of IFN-γ, there is added an amino acid to a concentration of not less than 1 mg/ml, preferably 5 to 50 mg/ml. The above-mentioned amino acid can also be added to said IFN-γ-containing aqueous solution in the process of its production. This amino acid-containing aqueous solution of IFN-γ can be submitted to the subsequent step with or without further addition of the amino acid to the above amino acid concentration as necessary.

Furthermore, the above-mentioned HSA or/and sugar and so forth can further be added.

When the pH adjustment is desired, the composition may be adjusted to the prescribed pH by adding mineral acid (hydrochloric acid, sulfuric acid, etc.) or/and in organic base (sodium hydrooxide, sodium carbonate, etc.).

The frozen human IFN-γ composition according to the invention can be produced, for example by freezing the above aqueous solution generally at -80° to -30°C. Said frozen composition is preferably stored at -80° to -10°C.

The lyophilized human IFN-γ composition according to the invention can be produced, for example by drying the above frozen composition in the conventional manner under reduced pressure or, as desired, by distributing an aqueous solution obtained by thawing the above frozen composition into containers, then freezing the solution in the same manner as above and finally drying the frozen matter in the conventional manner under reduced pressure.

In producing the lyophilized human IFN-γ composition according to the invention as an injectable preparation, it is preferable to purify, prior to distribution in portions, the aqueous solution of said composition or the components thereof by filtration for removing possibly occurring microorganisms, for instance, distribute the solution aseptically into vials or the like and then subject the same to the above lyophilization.

The frozen or lyophilized human IFN-γ composition according to the invention is advantageous in that the decrease in IFN-γ activity or deterioration in quality during the process of freezing or lyophilization and during storage is very slight and that no turbidity is produced upon its redissolution. Moreover, the lyophilized composition is obtained as a stabilized human IFN-γ powder, which can be used with advantage as a preparation for parenteral administration, among others.
In this case the composition to which HSA is further added, the adsorption thereof on the container wall is effectively prevented.

For use as an injectable preparation, the lyophilized human IFN-γ composition according to the invention is dissolved, generally at the time of use, in 1-100 ml/vial of distilled water for injection or glucose injection, for instance and the solution is used as far as the osmotic pressure thereof is physiologically acceptable. The lyophilized composition can also be used in the dosage form suited for ocular, otic or nasal administration as prepared by using an appropriate carrier, excipient and/or diluent.

The frozen or lyophilized human IFN-γ composition according to the invention is low in toxicity and can be used for the same purposes and in the same manner as the known human IFN-γ species.

In the present specification, the IFN-γ activity (antiviral activity) was determined in terms of international units (IU) in the following manner.

An international standard IFN-γ with its potency in units established and a sample to be assayed were tested for the ability to inhibit the cytopathic effect of Sindbis virus on the human amnion-derived FL cell line and the potency in question was determined by calculation based on the potency ratio found.

The protein amount in solution was estimated on the assumption that E:280 nm = 1.0 is equivalent to 1 mg.

## BRIEF DESCRIPTION OF THE DRAWING

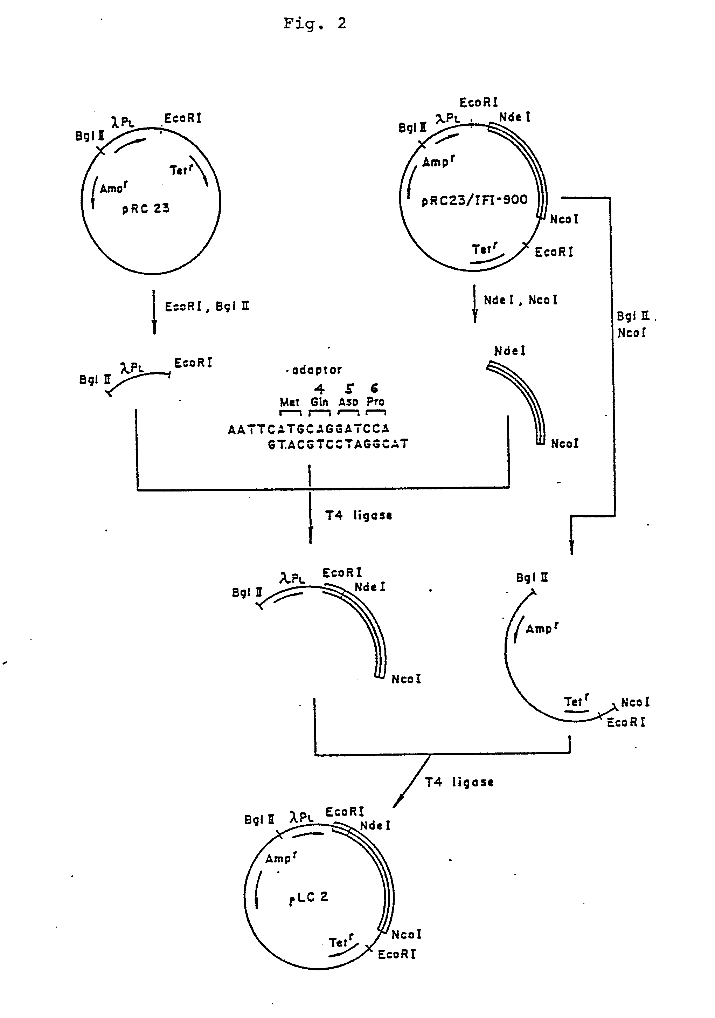

Fig. 1 shows an example of the amino acid sequence of IFN-γ composed of 146 amino acids.  Fig.2 shows the contruction scheme of plasmid pLC2 disclosed in reference example 6(i).

The following examples are further illustrative of the present invention.  However, they are by no means limitative of the scope of the invention.

In the examples, the high concentration aqueous solution of human rIFN-γ which was produced by the method described in Reference Example 1 and was substantially free of inorganic salts was used as the source of IFN-γ, unless otherwise specifically indicated. Said human IFN-γ has the amino acid sequence given in Fig. 1.

As tne production of the compositions disclosed in Examples 1-8, the pH ranges of them were pH 5.7-7 when the compositions were reconstituted with distilled water for injection, through they were not constructivly adjusted the pH.

Example 1

To an aqueous human IFN-γ solution (1 ml) having a potency of $2.4 \times 10^6$ IU/ml and containing 3 mg of glutathione (reduced form) as obtained after sterile filtration, there was added an aqueous solution (0.5 ml) containing 15 mg of glycine as obtained after sterile filtration, and the mixture was lyophilized in a vial. The lyophilizate was reconstituted with 1 ml of distilled water for injection and the solution was evaluated by visual inspection for clarity and assayed for IFN-γ potency. The results obtained are shown in Table 1.

Example 2

The procedure of Example 1 was followed except that 37.5 mg of hydroxyethylstarch (average molecular weight 200,000) was used together with 15 mg of glycine. The results obtained are shown in Table 1.

Example 3

The procedure of Example 1 was followed except that the amount of glycine was increased to 30 mg and further 7.5 mg of sodium glutamate was added. The results obtained are shown in Table 1.

Example 4

To an aqueous human IFN-γ solution (1 ml) having a potency of $3.7 \times 10^6$ IU/ml and containing 3 mg of glutathione (reduced form) as obtained after sterile filtration, there was added an aqueous solution containing 30 mg of glycine as obtained after sterile filtration, and the mixed solution was lyophilized in a vial. The lyophilizate was reconstituted with 1 ml of distilled water for injection and the solution was evaluated by visual inspection for clarity and assayed for IFN-γ potency.

In a control run in which the addition of glycine was omitted, an aqueous human IFN-γ soltuion (1 ml) containing 3 mg of glutathione (reduced form) as obtained after sterile filtration was lyophilized in a vial and the IFN-γ potency determination was carried out in the same manner. The results obtained are shown in Table 1.

- 13 -

0168008

Table 1

| Example | Clarity of Solution | Residual potency* |
|---|---|---|
| 1 | Clear | 100% |
| 2 | Clear | 94% |
| 3 | Clear | 104% |
| 4 | Clear | 98% |
| 4 (Control) | Clear | 86% |

*: Percentage of residual potency in lyophilizate based on potency in solution before lyophilization

Example 5

To an aqueous human IFN-γ solution (1 ml) having a potency of $4.6 \times 10^6$ IU/ml and containing 3 mg of glutathione (reduced form) as obtained after sterile filtration, there was added an aqueous solution (0.5 ml) containing 30 mg of glycine as obtained after sterile filtration. The mixed solution was stored in the frozen state at -30°C for 1 week and then tested for IFN-γ potency. The frozen composition exhibited a potency of 97% as compared with the solution before freezing.

Example 6

To an aqueous human IFN-γ solution (0.25 ml; $4.6 \times 10^6$ IU/ml; containing 3 mg/ml of glutathione (reduced form) and 2.5 mg/ml of sodium chloride) prepared by sterile filtration of the rIFN-γ-containing solution obtained in Reference Example 2, there was added an aqueous solution (0.75 ml) containing 15 mg of glycine as obtained after sterile filtration, and the mixed solution was lyophilized in a vial. The lyophilizate was reconstituted with 1 ml of distilled water for injection, whereupon a slight amount of fine insoluble matters was observed. The solution in that state was subjected to IFN-γ potency assay.

The potency found was 98% of that of the human IFN-γ solution before lyophilization.

Example 7

To an aqueous human IFN-γ solution (0.5 ml; $2.6 \times 10^6$ IU/ml; containing 3 mg/ml of glutathione (reduced form)) obtained after sterile filtration, there was added an aqueous solution (0.25 ml) containing 10 mg of glycine and 15 mg of hydroxyethylstarch (average molecular weight 40,000) as obtained after sterile filtration, and the mixed solution was lyophilized in a vial. The lyophilizate was reconstituted with 0.5 ml of distilled water for injection and the solution, after confirmation of its being clear , was tested for IFN-γ potency.

The potency found was 94% of that of the human IFN-γ solution before lyophilization. The residual potency after storage at 40° for 1 month was 100% based on the potency at the start of the storage, demonstrating its being stable

Example 8

To an aqueous human IFN-γ solution containing 3 mg/ml of glutathione (reduced form) (0.5 ml;

2.6 x $10^6$ IU/ml) obtained after sterile filtration, there was added an aqueous solution (0.25 ml) containing 15 mg of sodium glutamate and 15 mg of hydroxyethylstarch (average molecular weight 40,000) as obtained after sterile filtration, and the mixed solution was lyophilized in a vial. The lyophilizate was reconstituted with 0.5 ml of distilled water for injection and the solution, after confirmation of its being clear, was tested for IFN-γ potency.

The potency thus found was 102% based on that of the human IFN-γ solution before lyophilization. The residual potency after one-month storage at 40° was 106% based on the potency at the start of storage, demonstrating its being stable.

The use of the substantially inorganic salt-free, high concentration aqueous solutions of IFN-γ as obtained in Reference Examples 3-5 gives substantially the same results as those mentioned in the above examples.

Example 9

An aqueous solution comprising 0.16 ml/ml of an aqueous solution of human IFN-γ in a concentration of 3.8 x $10^6$ IU/ml containing 3 mg/ml of glutathione (reduced form), 5 mg/ml of HSA and 23 mg/ml of glycine, adjusted to pH 4.5 with 0.1 N HCl and aseptically filtered was distributed, in 1-ml portions, into vials and lyophilized. The lyophilizate in each vial was reconstituted with 1 ml of distilled water for injection and the solution was examined for clarity by visual inspection and submitted to pH measurement and IFN-γ activity assay. The results obtained are shown in Table 2.

Example 10

The procedure of Example 9 was followed except that the concentration of HSA in the aqueous solution was increased to 10 mg/ml. The results thus obtained are shown in Table 2.

Example 11

The procedure of Example 9 was followed except that the concentration of HSA in the aqueous solution was increased to 20 mg/ml. The results obtained are shown in Table 2.

Example 12

The procedure of Example 9 was followed except that hydroxyethylstarch (average molecular weight: 40,000) was further added to make a concentration of 5 mg/ml. The results obtained are shown in Table 2.

Example 13

The procedure of Example 10 was followed except that sodium glutamate was used in lieu of glycine, in an amount of 27 mg/ml, and that the aqueous solution was adjusted to pH 7.8 with 0.1 N NaOH.    The results obtained are shown in Table 2.

Table 2

| Example | Clarity of solution | pH | Residual potency* |
|---|---|---|---|
| 9 | Clear | 4.5 | 107% |
| 10 | Clear | 4.4 | 105% |
| 11 | Clear | 4.4 | 98% |
| 12 | Clear | 4.6 | 109% |
| 13 | Clear | 7.5 | 95% |

* Percent residual potency of the lyophilizate as compared with the solution before lyophilization

The use of the substantially inoranic salt-free, high-concentration aqueous solutions of rIFN-γ as obtained in Reference Examples 3-5 also gives substantially the same results as those obtained in the above examples.

Example 14

An aqueous solution comprising 0.25 ml/ml of an aqueous solution of des(Cys-Tyr-Cys)IFN-γ in a concen-

tration of $2.5 \times 10^6$ IU/ml as obtained by the procedure of Reference Example 6, 5 mg/ml of HSA and 23 mg/ml of glycine, adjusted to pH 4.5 with 0.1 N HCl and aseptically filtered was distributed, in 1-ml portions, into vials and lyophilized.

The lyophilizate in each vial was reconstituted with 1 ml of distilled water for injection. The solution was clear and had a pH of 4.5. The residual potency as compared with the potency of the aqueous solution before lyophilization was 96%.

Example 15

An aqueous solution comprising 0.25 ml/ml of an
aqueous solution of des(Cys-Tyr-Cys)IFN-γ in a concen-
tration of $2.5 \times 10^6$ IU/ml as obtained by the procedure of Reference Example 6, 15 mg/ml of hydroxyethylstarch (average molecular weight: 40,000) and 23 mg/ml of glycine and aseptically filtered was distributed, in 1-ml portions, into vials and lyophilized.

Upon reconstitution in the same manner as Example 14, the solution appeared clear and showed a pH of 6.5. The residual potency as compared with the potency of the aqueous solution before lyophilization was 101%.

Reference Example 1  Production of substantially inorganic
salt-free, high concentration aqueous
solution of rIFN-γ, I

(I)  To 1,000 g of frozen cells obtained by cultivating the strain Escherichia coli (RRI (pRK248cIts,pRC231/IFI-990) carring the human IFN-γ gene for expression in accordance with the description in Example 8 in the specification of EPC patent publication (laid open) No. 0 089 679, there was added 3,000 ml of 100 mM Tris-hydrochloric acid buffer (pH 7.0) containing 7 M guanidine hydrochloride and 2 mM phenyl-methylsulfonyl fluoride, and the mixture was stirred at 4°C for 1 hour and then centrifuged (17,000 rpm/30 minutes). The thus-obtained, clear and transparent supernatant was diluted 70-fold with a buffer containing 137 mM sodium chloride, 27 mM potassium chloride, 8 mM disodium hydrogen phosphate and 147 mM potassium phosphate (said buffer being hereinafter referred to as PBS). The resultant precipitate was removed using a Sharples centrifuge (10,000 rpm). The supernatant obtained (220 liters) was concentrated to 15 liters using a Pericon membrane filter (Millipore; permeation limit molecular weight: 10,000). The concentrate was allowed to stand at 4°C overnight and the resultant precipitate was removed by using a Sharples centrifuge again. The supernatant was applied to a previously packed 5 x 30 cm antibody column [Ab(Mo·Y2-11.1); cf. Example 12 in the specification of EPC (laid open) 0 103 898] at a flow rate of 1,000 ml/hour. Then, 2,500 ml of PBS, 5,000 ml of 10 mM phosphate buffer (pH 7.0) containing 1 M sodium chloride and 0.1% Tween 20, 2,500 ml of PBS and 2,500 ml of 20 mM phosphate buffer (pH 7.0) containing 0.5 M guanidine hydrochloride were passed through the antibody column successively in that order for washing the column, followed by elution with 20 mM phosphate buffer (pH 7.0) containing 2 M guanidine hydrochloride, which gave 500 ml of an eluate fraction having antiviral activity.

(II)  To a 420-ml portion of the eluate fraction obtained
by the procedure of Reference Example 1 (I), there was added
glutathione (reduced form) to a concentration of 10 mM.
This aqueous human IFN-γ solution (420 ml) was applied to
a Sephacryl S-200 (Pharmacia) column (9 x 100 cm) equilibrated
in advance with 25 mM acetate buffer (pH 6.0) containing 1
mM ethylenediaminetetraacetate, 150 mM sodium chloride, 10
mM glutathione (reduced form) and 2 M guanidine hydrochloride.
Elution with the same buffer gave 450 ml of a monomer eluate
fraction.  The above procedure gave an IFN-γ solution (0.410
mg/ml) with a specific activity of 3.4 x 10$^6$ IU/mg protein.
(III)  To the human IFN-γ (monomer) eluate fraction (450 ml)
obtained in Reference Example 1 (II), there was added, as a
diluent, 3,240 ml of 25 mM acetate buffer (pH 6.0) containing
10 mM glutathione (reduced form), 150 mM sodium chloride,
0.5 M guanidine hydrochloride and 0.01% Tween 20.  The thus-
prepared low concentration solution (protein content 0.05
mg/ml) was applied to a Sephadex G-25 column (14 x 100 cm)
equilibrated in advance with 25 mM acetate buffer (pH 6.0)
containing 10 mM glutathione (reduced form), 150 mM sodium
chloride and 0.01% Tween 20, followed by effecting gel
filtration with the same buffer.  There was obtained 3,180
ml (155.8 mg) of a guanidine hydrochloride-free human IFN-γ
eluate fraction.  The protein content in this fraction was
0.049 mg/ml.  The potency recovery was 84.4% and the specific activity was $3.5 \times 10^6$ IU/mg protein. This fraction was aged at 4°C for 48 hours and then concentrated to 159 ml by ultrafiltration using a Diaflo PM-10, 43 mm ⌀ (Amicon's ultrafiltration membrane). The concentrate was clear and transparent and had a protein content of 0.92 mg/ml. The protein recovery was 93.9% (146.3 mg). The specific activity of human IFN-γ was $6.8 \times 10^6$ IU/mg protein.

(IV) A 38-ml portion of the aqueous solution obtained by the procedure of (III) above and containing human IFN-γ in a high concentration (protein content: 0.952 mg/ml) was applied to a Sephadex G-25 column (5.0 x 50.0 cm) equilibrated in advance with 25 mM acetate buffer (pH 6.0) containing 10 mM glutathione (reduced form), followed by development with the same buffer. Thus was obtained 57 ml of a clear and transparent IFN-γ solution substantially free of inorganic salts (less than 10 ppm) with a protein content of 0.589 mg/ml.

This IFN-γ had a specific activity of $3.7 \times 10^6$ IU/mg protein.

Reference Example 2   Preparation of high concentration
                     aqueous solution of rIFN-γ

A 38-ml portion of the aqueous solution obtained by the procedure of Reference Example 1 (III) and containing human IFN-γ in a high concentration (protein content: 0.952 mg/ml) was applied to a Sephadex G-25 column (5.0 x 50.0 cm) equilibrated in advance with 25 mM acetate buffer (pH 6.0) containing 10 mM glutathione (reduced form) and 40 mM sodium chloride, followed by development with the same buffer.
The thus-obtained clear and transparent IFN-γ solution
(52 ml) contained 0.04 M sodium chloride and the protein
content was 0.658 mg/ml.

The specific activity of this IFN-γ was $4.6 \times 10^6$
IU/mg protein.

Reference Example 3   Production of substantially inorganic
salt-free, high concentration aqueous
solution of rIFN-γ, II

(I)   To 1 kg of frozen cells obtained by cultivating the
transformant Escherichia coli 294/pHITtrp2101 described in
Reference Example 2 in the specification of EPC (laid open)
0 103 898, there was added 3,000 ml of 0.05 M borate buffer
(pH 7.2) containing 7 M guanidine hydrochloride. The mixture was stirred at 4°C for 1 hour and then centrifuged
(17,000 rpm/30 minutes) to give a clear and transparent
extract (2,700 ml). This extract was diluted 10-fold with
a buffer composed of 0.137 M sodium chloride, 2.7 mM potassium chloride, 8 mM disodium hydrogen phosphate and 1.47 mM
monopotassium phosphate (said buffer being hereinafter
referred to as PBS). To this dilution was added 0.4 kg
of silica gel (Separation Industries). After stirring at
4°C for 45 minutes, the mixture was allowed to stand for
15 minutes, and the supernatant was discarded by decantation. The silica gel was washed well with 0.05 M phosphate
buffer (pH 7.2) containing 1 M sodium chloride and packed
into a column (11 x 11 cm). Elution was carried out with 0.01 M borate buffer (pH 8.0) containing 0.5 M tetramethylammonium chloride and the eluate (20 liters) was applied
to a 5 x 8 cm antibody column [Ab(Mo·γ2-11.1); vide supra].
The antibody column was washed with 750 ml of PBS and then
eluted with 0.02 M phosphate buffer (pH 7.0) containing 2 M
guanidine hydrochloride to give an antivirally (hereinafter
abbreviated as AV) active fraction (183 ml). To this
eluate was added an amount (562 mg) of glutathione (reduced
form) to 0.01 M. Thus was obtained an aqueous solution
containing 172 mg of rIFN-γ with a specific activity of
$2.1 \times 10^6$ IU/mg.

(II)   The rIFN-γ-containing aqueous solution (183 ml)
obtained in Reference Example 3 (I) was applied to a
Sephacryl S-200 (pharmacia) column (9 x 79 cm) equilibrated
in advance with 25 mM acetate buffer (pH 6.0) containing
1 mM ethylenediaminetetraacetate, 0.15 M sodium chloride,
0.01 M glutathione (reduced form) and 2 M guranidine hydrochloride, followed by development with the same buffer.
There was collected a monomer eluate fraction (433 ml).
Sodium dodecyl sulfate-polyacrylamide gel slab electrophoresis (hereinafter abbreviated as SDS-PAGE) of the
fraction obtained in the above manner showed convergence
to the monomer. The above gel filtration treatment gave
an aqueous solution containing 142 mg of rIFN-γ with a
specific activity of $3.0 \times 10^6$ IU/mg protein.

(III)   To a 36.6 ml (12.0 mg) portion of the rIFN-γ-containing
aqueous solution obtained above in (II), there was added 163.4 ml of 25 mM acetate buffer (pH 6.0) containing 0.01 M glutathione (reduced form) and 0.5 M guanidine hydrochloride. The thus-prepared 0.06 mg/ml solution (200 ml) was applied to a Sephadex G-25 (Pharmacia) column (5 x 60 cm) equilibrated in advance with 25 mM acetate buffer (pH 6.0) containing 0.01 M glutathione (reduced form), followed by development and elution with the same buffer. The thus-obtained rIFN-γ fraction (220 ml) had an rIFN-γ concentration of 0.047 mg/ml. This solution was aged at 4°C for 2 days and then concentrated to 14 ml by ultrafiltration using a Diaflo PM-10 membrane (Amicon's ultrafiltration membrane) to give a clear and transparent solution of rIFN-γ (protein content 0.711 mg/ml) substantially free of inorganic salts (less than 10 ppm). The thus-obtained high concentration rIFN-γ converged to the monomer in SDS-PAGE. The specific acitivity of this rIFN-γ was $3.6 \times 10^6$ IU/mg protein.

Reference Example 4    Production of substantially inorganic salt-free, high concnetration aqueous solution of rIFN-γ, III

To a 35-ml (11.48 mg) portion of the aqueous rIFN-γ solution obtained in Reference Example 1 (II), there was added 165 ml of 25 mM acetate buffer (pH 6.0) containing 0.01 M glutathione (reduced form) and 0.5 M guanidine hydrochloride and the resulting 0.057 mg/ml solution (200 ml) was applied to a Sephadex G-25 column (5 x 60 cm) equilibrated in advance with 0.01 M glutathione (reduced form) solution (pH 6.0), followed by development with 0.01 M glutathione (reduced form) solution (pH 6.0). The thus-obtained rIFN-γ eluate fraction (235 ml) had an rIFN-γ concentration of 0.046 mg/ml. The solution was aged at 4°C for 1 day and concentrated to 10 ml by ultrafiltration using a Diaflo PM-10 membrane. There was obtained a clear and transparent aqueous solution of rIFN-γ (protein concentration 1.08 mg/ml) substantially free of inorganic salts (less than 10 ppm) and of acetate buffer. The thus-obtained rIFN-γ converged to the monomer in SDS-PAGE. The specific activity of this rIFN-γ was $3.6 \times 10^6$ IU/mg protein.

Reference Example 5   Production of substantially inorganic salt-free, high concentration aqueous solution of rIFN-γ, IV

A 35-ml (11.48 mg) portion of the rIFN-γ solution obtained in Reference Example 1 (II) was diluted by adding thereto 165 ml of 25 mM acetate buffer (pH 6.0) containing 0.01 M glutathione (reduced form) and 0.5 M guanidine hydrochloride to give 200 ml of a solution having a concentration of 0.057 mg/ml. This solution was applied to a Sephadex G-25 column (5 x 60 cm) equilibrated in advance with a solution (pH 6.0) containing 0.267 M glycine and 0.01 M glutathione (reduced form). Development with the same solution gave an rIFN-γ eluate fraction (220 ml). This solution had an rIFN-γ concentration of 0.046 mg/ml. The solution was aged at 4°C for 2 days and then concentrated to 9.4 ml by ultrafiltration using a Diaflo PM-10 membrane to give a clear and transparent rIFN-γ solution (protein content 1.07 mg/ml) substantially free of inorganic salts (less than 10 ppm) and of acetate buffer. This rIFN-γ converged to the monomer in SDS-PAGE and its specific acitivity was 3.62 x $10^6$ IU/mg protein.

Reference Example 6  Production of substantially inorganic salt
salt-free, high concentration aqueous solution of des (Cys-$^1$
Tyr-$^2$Cys-$^3$) IFN-γ

(i)  Transformant production

The IFN-γ expression plasmid pRC23/IFI-900 [cf.
Example 7 of the specification for a patent application
under EPC as laid open under No. 0089676] was digested
with the restriction enzymes NdeI and NcoI, and a 710 bp
NdeI-NcoI DNA fragment (A) containing the IFN-γ gene region
was isolated.  Separately, the plasmid pRC23 was digested
with the restriction enzymes BglII and EcoRI, and a 265 bp
DNA fragment (B) containing the λP$_L$ promoter was isolated.
The fragments (A) and (B) and the chemically synthesized,
protein synthesis start codon-containing oligonucleotide

AATTCATGCAGGATCCA

GTACGTCCTAGGTAT

were joined together using T4 DNA ligase, with the NdeI
and EcoRI cohesive ends as the sites of joining.  The DNA
fragment thus obtained was joined to the plasmid pRC23/IFI-
900 after treatment with NcoI and BglII, to thereby con-
struct an expression plasmid, pLC2, coding for the Cys-$^1$
Tyr-$^2$Cys-$^3$-deficient IFN-γ polypeptide (Fig. 2).  This plasmid
pLC2 was used for transforming Escherichia coli RRI(pRK248
cIts) by the method of Cohen et al.[P.N.A.S. (USA), 69,
2110 (1972)] to give a transformant, Escherichia coli
(=E. coli) PRI(pLC2,pRK248 cIts).

(ii)  Transformant cultivation

The strain E. coli RRI(pLC2,pRK248 cIts) carrying
the plasmid constructed in (i) above was shake-cultured at 35°C in 50 ml of a liquid medium containing 1% Bacto-tryptone, 0.5% yeast extract, 0.5% sodium chloride and 7 μg/ml tetracycline. The culture broth was transferred to 2.5 liters of M9 medium containing 0.5% casamino acids, 0.5% glucose and 7 μg/ml tetracycline, and grown at 35°C for 4 hours and then at 42°C for 3 hours. Cells were harvested by centrifugation and stored at -80°C.

(iii) Purification

In 22 ml of 0.1 M Tris-hydrochloride buffer (pH 7.0) containing 7 M guanidine hydrochloride and 2 mM phenyl-methylsulfonyl fluoride, there were suspended 7.1 g of frozen cells obtained in the same manner as mentioned above in (ii). The suspension was stirred at 4°C for 1 hour and then centrifuged at 10,000 x g for 30 minutes to give 24 ml of a supernatant. This supernatant was diluted by adding 300 ml of PBS and the dilution was applied to an antibody column (Moγ2-11.1, column capacity 15 ml) at a flow rate of 1 ml/minute. Thereafter, the column was washed with 60 ml of 20 mM sodium phosphate buffer (pH 7.0) containing 0.5 M guanidine hydrochloride and then eluted with 45 ml of 20 mM sodium phosphate buffer (pH 7.0) containing 2 M guanidine hydrochloride, to give 25 ml of an antivirally active fraction. This fraction (25 ml) was applied to a Sephacryl S-200 (Pharmacia) column (2.6 x 94 cm; column capacity 500 ml) equilibrated in advance with 25 mM ammonium acetate buffer (pH 6.0) containing 1 mM ethylenediaminetetraacetic acid, 0.15 M sodium chloride, 10 mM cysteine and 2 M guanidine hydrochloride, and eluted with the same buffer to give 40 ml of an antivirally active fraction.

The thus-obtained Cys-Tyr-Cys-deficient IFN-γ polypeptide des(Cys-Tyr-Cys) IFN-γ weighed 7.0 mg and had a specific activity of $2.7 \times 10^6$ IU/mg.

(iv)   Production of high-concnetration aqueous solution

A 2.2-ml portion of the des(Cys$^1$-Tyr$^2$-Cys$^3$)IFN-γ-containing eluate fraction (protein concentration: 0.18 mg/ml) obtained above in (iii) was diluted with 17.6 ml of 25 mM ammonium acetate buffer (pH 6.0) containing 0.5 M guanidine hydrochloride.   The dilution was loaded onto a Sephadex G-25 column (2.6 x 15 cm) equilibrated in advance with 25 mM ammonium acetate buffer (pH 6.0). The subsequent elution with the same buffer gave 23 ml of a des(Cys$^1$-Tyr$^2$-Cys$^3$)IFN-γ-containing eluate fraction (protein concentration: 0.016 mg/ml) freed of guanidine hydrochloride.

After 24 hours of aging at 4°C, the eluate was concentrated by ultrafiltration using a Diaflo-YM-10 (25 mm ⌀, Amicon) and filtered through a filter (0.2 μm) to give 0.68 ml of a clear solution having a protein concentration of 0.41 mg/ml.

What is claimed is:

1. A human γ-interferon composition frozen or lyophilized in the substantial absence of inorganic salt and in the presence of amino acid.

2. The composition according to claim 1, wherein the amino acid is a monoamino aliphatic amino acid.

3. The composition according to claim 1, wherein the human γ-interferon is a recombinant human γ-interferon.

4. The composition according to claim 3, wherein the recombinant human γ-interferon is originated highly concentrated aqueous recombinant human γ-interferon solution.

5. The composition according to claim 1, wherein the human γ-interferon has a specific activity of $1 \times 10^5$ to $1 \times 10^7$ IU/mg.

6. The composition according to claim 1, wherein the human γ-interferon is in a concentration of $1 \times 10^2$ to $1 \times 10^7$ IU/ml as an aqueous solution.

7. The composition according to claim 1, wherein the human γ-interferon is the polypeptide consisting of 146 amino acids of the sequence shown in Figure 1.

8. The composition according to claim 1, wherein the human γ-interferon is des(Cys$^1$-Tyr$^2$-Cys$^3$)IFN-γ.

9. The composition according to claim 1, wherein the amino acid is in a concentration of 5 to 50 mg per ml as an aqueous solution.

10. The composition according to claim 2, which contains human serum albumin in addition to the monoamino aliphatic amino acid.

11. The composition according to claim 10, wherein the human serum albumin is in a concentration of 2 to 20 mg per ml as an aqueous solution.

12. The composition according to claim 10, wherein the monoamino aliphatic amino acid is a neutral monoamino aliphatic amino acid.

13. The composition according to claim 12, wherein the neutral monoamino aliphatic amino acid is glycine.

14. The composition according to claim 12, which is adjusted as showing pH of 4.0 to 5.0 as an aqueous solution.

15. The composition according to claim 10, wherein the monoamino aliphatic amino acid is an acidic monoamino aliphatic amino acid.

16. The composition according to claim 15, which is adjusted as showing pH of 7.5 to 8.5 as an aqueous solution.

17. The composition according to claim 2 or 10, which further contains a sugar.

18. The composition according to claim 17, wherein the sugar is a polysaccharide.

19. The composition according to claim 17, wherein the sugar is in a concentration of 3 to 50 mg per ml as an aqueous solution.

20. The composition according to claim 1, which is a frozen form.

21. The composition according to claim 1, which is a lyophilized form.

22. A method of producing the composition defined in claim 1, which comprises adding an amino acid to a substantially inorganic salt-free aqueous solution containing human γ-interferon, freezing the resulting solution to produce the frozen composition and, if desired, drying the frozen composition under the reduced pressure to produce the lyophilized composition.

23. A method for stabilizing human γ-interferon, which comprises adding an amino acid to a substantially inorganic salt-free aqueous solution containing human γ-interferon, freezing the resulting solution and, if desired, drying the resulting frozen composition under the reduced pressure.

Fig. 1

```
1
Cys Tyr Cys Gln Asp Pro Tyr Val Lys Glu
                                        20
Ala Glu Asn Leu Lys Lys Tyr Phe Asn Ala

Gly His Ser Asp Val Ala Asp Asn Gly Thr
                                        40
Leu Phe Leu Gly Ile Leu Lys Asn Trp Lys

Glu Glu Ser Asp Arg Lys Ile Met Gln Ser
                                        60
Gln Ile Val Ser Phe Tyr Phe Lys Leu Phe

Lys Asn Phe Lys Asp Asp Gln Ser Ile Gln
                                        80
Lys Ser Val Glu Thr Ile Lys Glu Asp Met

Asn Val Lys Phe Phe Asn Ser Asn Lys Lys
                                        100
Lys Arg Asp Asp Phe Glu Lys Leu Thr Asn

Tyr Ser Val Thr Asp Leu Asn Val Gln Arg
                                        120
Lys Ala Ile His Glu Leu Ile Gln Val Met

Ala Glu Leu Ser Pro Ala Ala Lys Thr Gly
                                        140
Lys Arg Lys Arg Ser Gln Met Leu Phe Arg
                146
Gly Arg Arg Ala Ser Gln
```

Fig. 2